# EUROPEAN PATENT APPLICATION

(11) **EP 2 638 868 A1**
(43) Date of publication of application: **18.09.2013**
(21) Application number: 11839105.1
(22) Date of filing: 07.11.2011
(51) Int. Cl.: A61B 17/11, A61B 17/00, A61M 35/00, B05B 7/08

(54) **TOOL FOR APPLYING BIOLOGICAL TISSUE ADHESIVE**

(30) Priority: 10.11.2010 JP 2010251515
(71) Applicant: Sumitomo Bakelite Co., Ltd., Shinagawa-ku Tokyo 140-0002 (JP); The Chemo-Sero-Therapeutic Research Institute, Kumamoto-shi, Kumamoto 860-8568 (JP)
(72) Inventor: SUZUKI, Zenetsu, Akita-shi Akita 011-8510 (JP); IKEDA, Masao, Akita-shi Akita 011-8510 (JP); UCHIDA, Takanori, Kumamoto-shi Kumamoto 8608568 (JP); ICHIKI, Osamu, Kumamoto-shi Kumamoto 8608568 (JP); OKA, Shirou, Kumamoto-shi Kumamoto 8608568 (JP)
(74) Representative: Schnappauf, Georg
(86) International application number: PCT/JP2011/006222
(87) International publication number: WO 2012/063464

(57) **Abstract**

An adhesive applicator for biological tissue (100) includes a nozzle body (1), a gas inlet (2), medical fluid inlets (3a, 3b), medical fluid outlets (4a, 4b), medical fluid tubes (5a, 5b), a gas outlet (6), communication paths (8a, 8b), and a check valve (7b). The gas outlet (6) is located close to the medical fluid outlets (4a, 4b), and configured to eject gas loaded through the gas inlet (2) in the nozzle body (1) to thereby atomize medical fluids and mix them together. The communication paths (8a, 8b) communicate between inside of the nozzle body (1) and inside of the medical fluid tube (5b) . The check valve (7b) is located between the communication paths (8a, 8b) and the medical fluid inlet (3b). The check valve (7b) only allows one-way flow of the medical fluid from the medical fluid inlet (3b) into the medical fluid tube (5b) .

## Description

### [TECHNICAL FIELD]

The present invention relates to an adhesive applicator for biological tissue to be used for spraying a plurality of medical fluids at the same time for tissue adhesion onto an affected part of an organism, thus applying the medical fluids thereto.

### [BACKGROUND ART]

Adhesive applicators for biological tissue including a pair of medical fluid flow paths each including a syringe body, a medical fluid tube and a nozzle, are widely employed. Typically, fibrinogen solution and thrombin solution are respectively loaded in the syringe bodies. A fluid application method including spraying the medical fluids through the nozzles at the same time utilizing aseptic gas and mixing the medical fluids is called a spray application method. With the spray application method, the fibrinogen solution reacts with the thrombin solution to thereby turn into fibrin, which is a fibrous substance. The fibrous fibrin checks bleeding and covers an affected part, for example a damaged alimentary canal. In this process, the medical fluids may be intermittently sprayed, in other words the spraying may be temporarily suspended and then restarted. In such a case, the misty fine particles of the sprayed fibrinogen solution and thrombin solution may stick again to the tip portion of the nozzle during the suspension period, and the two fluids may contact each other. This leads to the drawback in that the fibrin solidifies on the outlet of the medical fluid and the distal end portion of the medical fluid tube, thus clogging the nozzle.

To avoid such clogging, the patent document 1 proposes providing a switching device in an aseptic gas inlet so as to allow the aseptic gas to flow through either the gas flow path or the medical fluid flow path. With such a configuration, when the dispensation of the medical fluid is suspended, the flow path of the aseptic gas is switched to the medical fluid flow path so as to eject the aseptic gas therethrough, and to discharge the medical fluid residing on the distal end portion of the flow path. A check valve is provided between the syringe and the medical fluid tube, to only allow one-way flow of the medical fluid into the medical fluid tube and inhibit a reverse flow. Accordingly, the medical fluid is prevented from flowing back into the syringe when the aseptic gas is discharging the residual medical fluid. However, the configuration according to the patent document 1 requires providing the switching device, and the physician has to operate the switching device when the dispensation of the medical fluid is suspended. On the other hand, the patent document 2 proposes forming a communication path through the wall of the medical fluid tube. The communication path is a slit obliquely formed in the tube wall. The depthwise direction of the slit is inwardly inclined toward a downstream side (dispensing side) from an upstream side (introduction side) of the medical fluid. The communication path is closed by the dispensing pressure during the dispensation of the medical fluid, however upon suspending the dispensation of the medical fluid the communication path is opened by the pressure of the aseptic gas, and the aseptic gas flows into the tube. Therefore, the aseptic gas discharges the medical fluid residing on the distal end portion of the tube thereby preventing clogging. Thus, the nozzle clogging can be prevented with a simplified structure, despite performing intermittent spraying.

### [RELATED DOCUMENTS]

### [PATENT DOCUMENT]

[Patent Document 1] JP-A-No.2001-238887
[Patent Document 2] JP-A-No.2007-252880

### [DISCLOSURE OF THE INVENTION]

### [Problem to be Solved by the Invention]

In recent years, however, there is a growing demand for further improvement in prevention of solidification of the nozzle of the applicator. Accordingly, the present invention provides an adhesive applicator for biological tissue used for spraying and mixing a plurality of medical fluids to obtain an adhesion effect, configured to more effectively prevent solidification of the medical fluids on the nozzle after spraying.

### [Solution to Problem]

According to the present invention, there is provided an adhesive applicator for biological tissue including a nozzle body having a space therein, a gas inlet through which gas is introduced into the space, a first medical fluid inlet and a second medical fluid inlet provided to the nozzle body, a first medical fluid outlet and a second medical fluid outlet provided at a distal end portion of the nozzle body, a first medical fluid tube and a second medical fluid tube arranged inside the nozzle body, and a gas outlet. The first medical fluid tube communicates between the first medical fluid inlet and the first medical fluid outlet. The second medical fluid tube communicates between the second medical fluid inlet and the second medical fluid outlet. The gas outlet is located close to the first and the second medical fluid outlets, and configured to eject the gas loaded in the nozzle body, to thereby atomize the medical fluids respectively dispensed through the first and the second medical fluid outlets and mix the medical fluids together. The second medical fluid tube includes a communication path communicating between inside of the nozzle body and inside of the second medical fluid tube. The adhesive applicator for biological tissue includes a check valve provided upstream from the communication path to only allow one-way flow of the medical fluid from the second medical fluid inlet into the second medical fluid tube.

In the adhesive applicator for biological tissue thus configured, the high-pressure gas for atomizing the medical fluid flows into the second medical fluid tube through the communication path, when the internal pressure in the medical fluid tube falls below a predetermined threshold. Since the check valve is provided upstream from the communication path, the gas flows toward the medical fluid outlet from the communication path. Therefore, the medical fluid residing in the vicinity of the medical fluid outlet of the second medical fluid tube with the communication path (residual medical fluid) is discharged outside. As a result, solidification at the distal end portion of the second medical fluid tube can be effectively prevented, during intermittent use of the applicator. Because of the presence of the check valve, the pressure of the gas is kept from being wasted upstream from the position where the check valve is located, but exclusively utilized for discharging the residual medical fluid. Such a configuration further ensures the prevention of the solidification at the distal end portion of the second medical fluid tube. In addition, the applicator having such high capability of preventing the solidification can be easily manufactured, simply by forming the communication path and providing the check valve.

In the adhesive applicator for biological tissue according to the present invention, the second medical fluid tube may include a plurality of communication paths formed at different positions in the axial direction of the second medical fluid tube.

In the adhesive applicator for biological tissue according to the present invention, the first medical fluid tube may include a communication path communicating between inside of the nozzle body and inside of the first medical fluid tube. In addition, aperture area of the communication path formed in the first medical fluid tube may be smaller than aperture area of the communication path formed in the second medical fluid tube. Here, the aperture area of the communication path refers to the total of the aperture area of zero pieces, one piece, or a plurality of pieces of communication paths. In the case where the first medical fluid tube is without the communication path, the aperture area of the communication path in the first medical fluid tube is zero. Thus, the first and the second medical fluid tubes may each include one or a plurality of communication paths, in which case the total of the aperture area of the communication path (s) in the second medical fluid tube may be larger than the total of the aperture area of the communication path(s) in the first medical fluid tube. Alternatively, the first medical fluid tube may remain without the communication path, and exclusively the second medical fluid tube may include the communication path.

The adhesive applicator for biological tissue according to the present invention may further include a first syringe body connected to the first medical fluid inlet and a second syringe body connected to the second medical fluid inlet, and the first medical fluid loaded in the first syringe body may have higher viscosity than the second medical fluid loaded in the second syringe body.

The adhesive applicator for biological tissue according to the present invention may further include an identification member for distinction between the first medical fluid tube and the second medical fluid tube.

The adhesive applicator for biological tissue according to the present invention may further include a first piston that introduces the first medical fluid from the first syringe body into the first medical fluid tube, a second piston that introduces the second medical fluid from the second syringe body into the second medical fluid tube, and a joint member connecting the first piston and the second piston.

The adhesive applicator for biological tissue according to the present invention may further include a syringe body joint member that combines the first syringe body and the second syringe body, and a fixing member that fixes the nozzle body and the syringe body joint member together.

In the adhesive applicator for biological tissue according to the present invention, the communication path may be a slit formed in a wall of the medical fluid tube, and a depthwise direction of the slit may be inwardly inclined toward a distal end portion of the medical fluid tube.

In the adhesive applicator for biological tissue according to the present invention, the opening width of the slit on the outer surface of the tube may be larger than the opening width of the slit on the inner surface of the tube wall.

In the adhesive applicator for biological tissue according to the present invention, the communication path may serve as a gas flow path having a length longer than a wall thickness of the medical fluid tube, and a depthwise direction of the gas flow path may be inwardly inclined toward a distal end portion of the medical fluid tube.

In the adhesive applicator for biological tissue according to the present invention, the medical fluid tube may be formed of a soft plastic tube.

The adhesive applicator for biological tissue according to the present invention may include a plurality of nozzle bodies each having a space therein and respectively associated with a plurality of medical fluid tubes.

It is to be noted that the constituents of the present invention do not have to be individually independent, but may be configured such that a plurality of constituents constitutes a single member, a constituent is composed of a plurality of members, a constituent is a part of another constituent, a part of a constituent and a part of another constituent overlap, and so forth. Further, the term "upstream" herein refers to the side of the medical fluid inlet, through which the medical fluid is introduced into the medical fluid tube, and the term "downstream" refers to the side of the medical fluid outlet, through which the medical fluid in the medical fluid tube is outwardly dispensed.

### [Advantageous Effect of the Invention]

The adhesive applicator for biological tissue according to the present invention is capable of effectively preventing solidification at the tip portion of a nozzle after spraying and mixing a plurality of medical fluids, with a simplified configuration.

### [BRIEF DESCRIPTION OF THE DRAWINGS]

The above and other objects, features, and advantages of the present invention will become more apparent through preferred embodiments described hereunder with reference to the accompanying drawings.

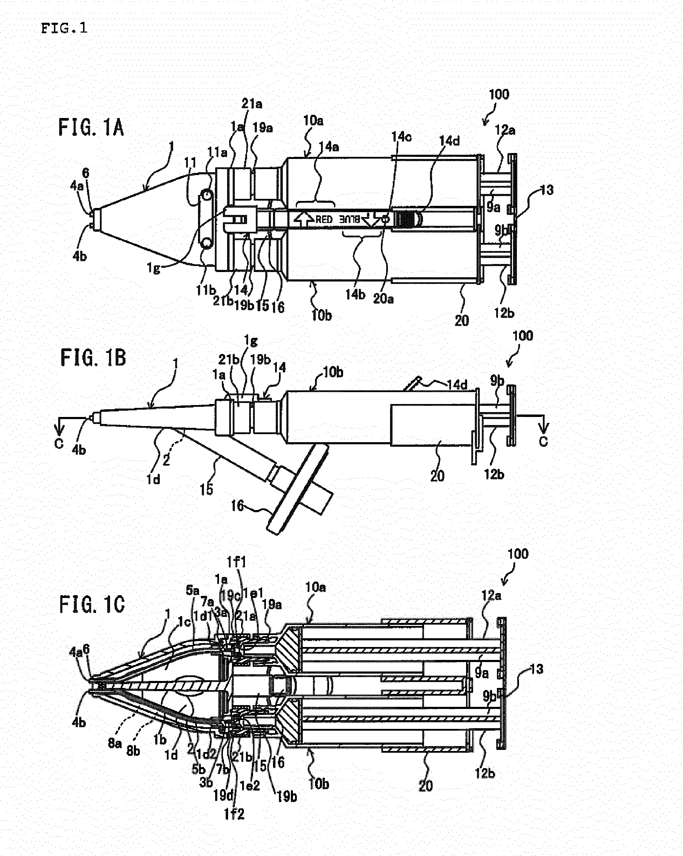

Figs. 1A to 1C represent an example of an adhesive applicator for biological tissue according to a first embodiment of the present invention, Fig. 1A being a plan view of the adhesive applicator viewed from the side of a band (fixing member), Fig. 1B being a side view of the adhesive applicator shown in Fig. 1A, and Fig. 1C being a cross-sectional view taken along a line C-C in Fig. 1B.

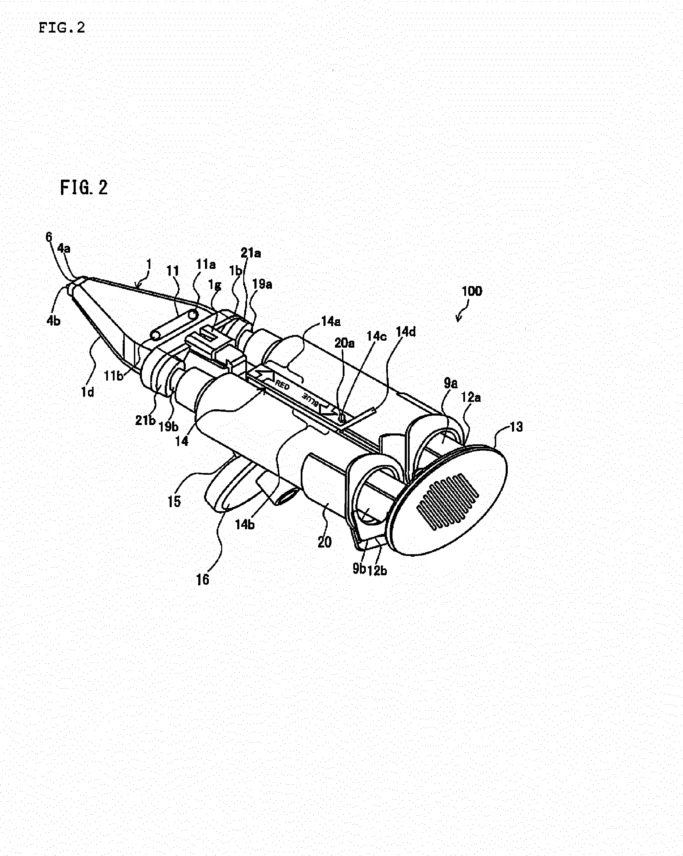

Fig. 2 is a perspective view showing the adhesive applicator for biological tissue according to the first embodiment.

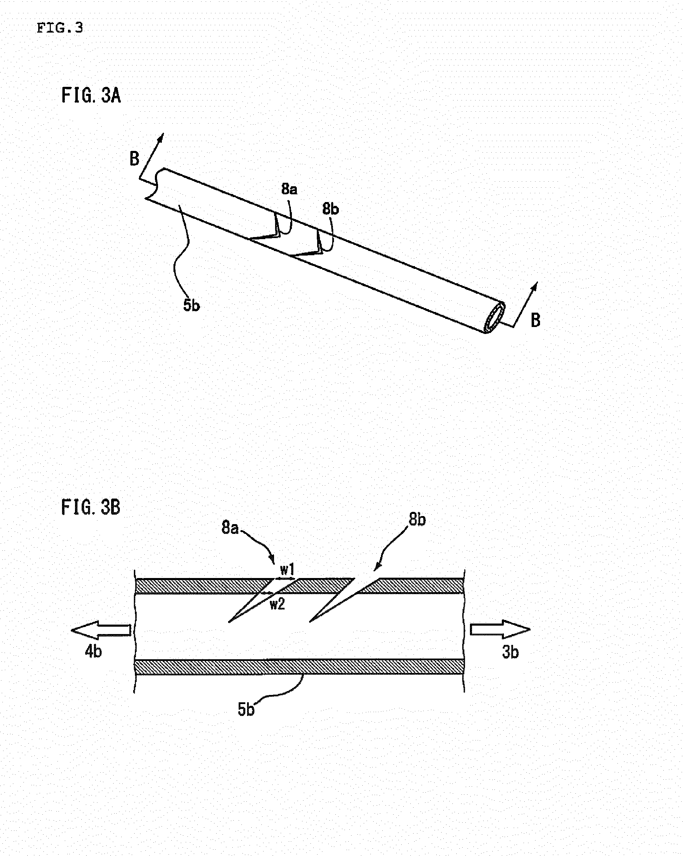

Fig. 3A is an enlarged fragmentary perspective view of a medical fluid tube with a slit-shaped communication path, in the adhesive applicator for biological tissue according to the first embodiment, and Fig. 3B is a cross-sectional view taken along a line B-B in Fig. 3A.

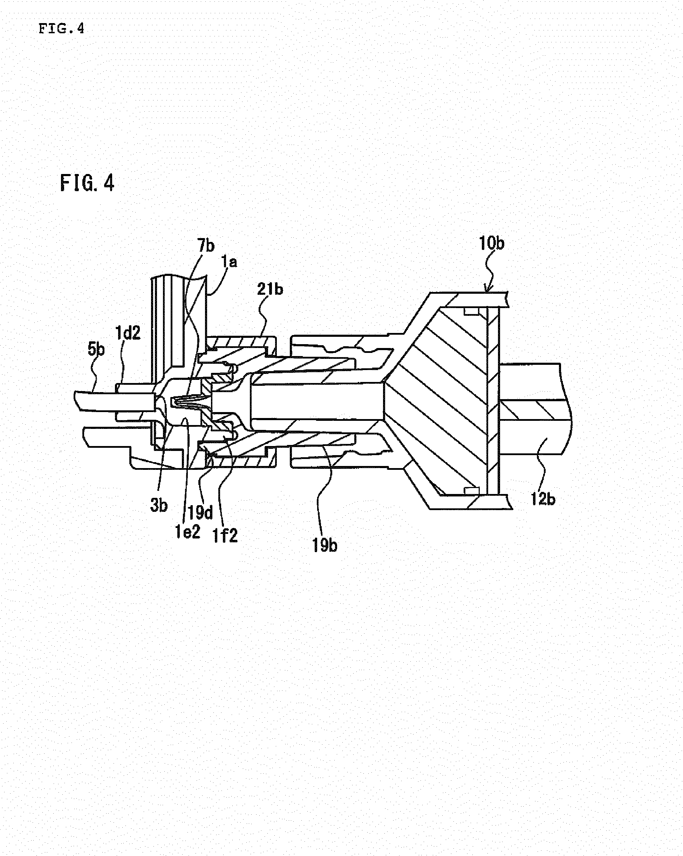

Fig. 4 is an enlarged fragmentary cross-sectional view of portion around a check valve in the adhesive applicator for biological tissue according to the first embodiment.

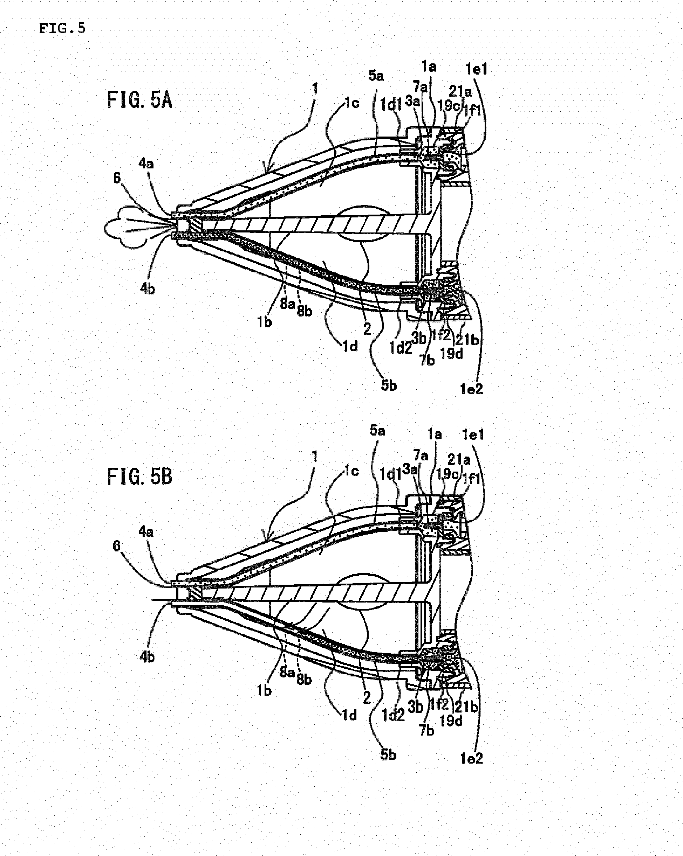

Figs. 5A and 5B are cross-sectional views of a part of the nozzle body of the adhesive applicator for biological tissue according to the first embodiment for explaining conditions of use thereof, Fig. 5A illustrating a state in which medical fluids are being dispensed, and Fig. 5B illustrating a state in which the dispensation of the medical fluids is suspended and the medical fluid is being discharged out of the medical fluid tube with the communication path.

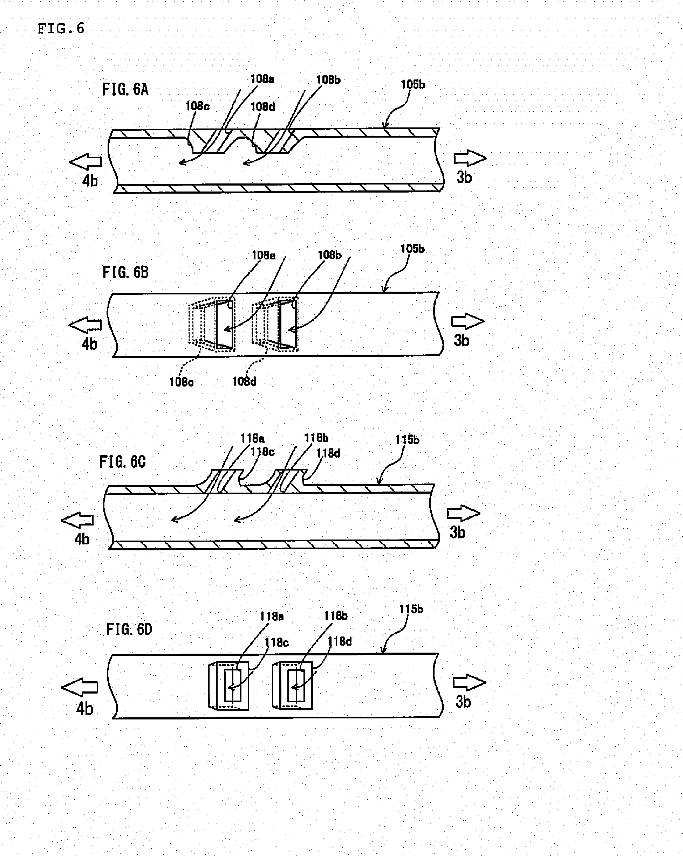

Figs. 6A to 6D represent an enlarged part of an adhesive applicator for biological tissue according to a second embodiment of the present invention, Fig. 6A being a cross-sectional view of the medical fluid tube including communication tubes connected to the inside of the communication path, Fig. 6B being a plan view of Fig: 6A, Fig. 6C being a cross-sectional view of the medical fluid tube where communication tubes are connected to the outside of the communication path, and Fig. 6D being a plan view of Fig. 6C.

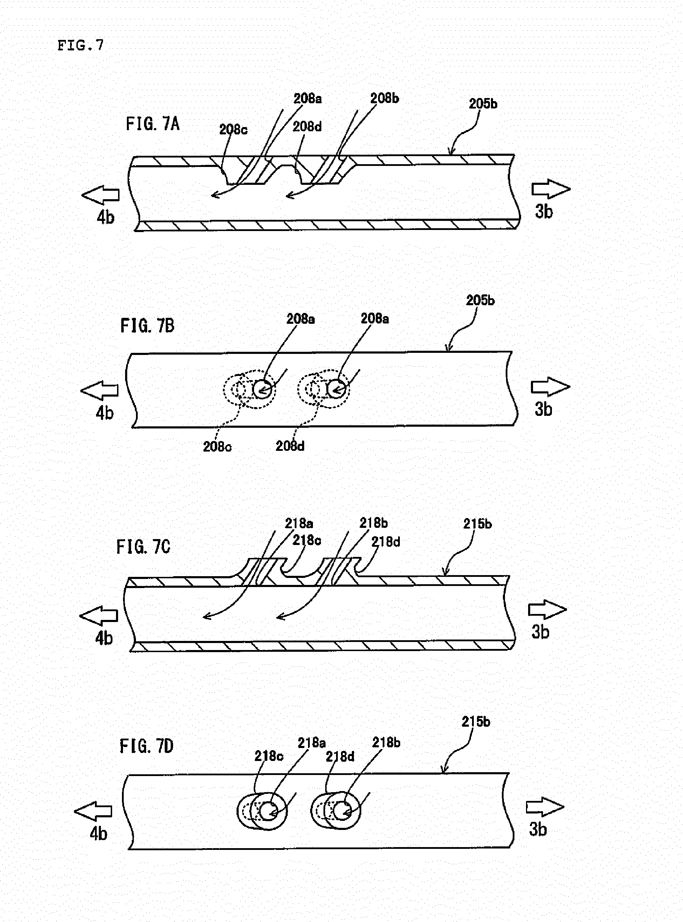

Figs. 7A to 7D represent an enlarged part of an adhesive applicator for biological tissue according to a third embodiment of the present invention, Fig. 7A being a cross-sectional view of the medical fluid tube where communication tubes are connected to the inside of the communication path, Fig. 7B being a plan view of Fig. 7A, Fig. 7C being a cross-sectional view of the medical fluid tube including communication tubes connected to the outside of the communication path, and Fig. 7D being a plan view of Fig. 7C.

### [DESCRIPTION OF EMBODIMENTS]

Hereafter, embodiments of the present invention will be described referring to the drawings. In all the drawings, the same constituents will be given the same numeral, and the description thereof will not be repeated.

In the embodiments, front and back, left and right, and up and downward directions will be specified on the basis of the drawings. However, it should be noted that those expressions are adopted merely for the sake of clarity in the description of the positional relationship between the constituents, and in no way intended to limit the direction in the manufacturing process of the product in which the present invention is implemented or in the use thereof.

### First Embodiment

Fig. 1A is a schematic plan view showing an example of an adhesive applicator for biological tissue, Fig. 1B is a side view of the adhesive applicator for biological tissue shown in Fig. 1A, and Fig. 1C is a cross-sectional view taken along a line C-C in Fig. 1B. The adhesive applicator for biological tissue according to this embodiment is suitably applicable to manual operation for hemostasis and closure of, for example, a surgical margin of a liver or a lung, or a sutured part of an alimentary canal. Figs. 1A to 1C illustrate a pair of syringe bodies respectively including different medical fluids, and connected to a nozzle body.

Referring to Figs. 1A to 1C, an outline of the adhesive applicator for biological tissue according to this embodiment will be described first. Hereinafter, an upward direction in Fig. 1B will be referred to as upward from the adhesive applicator for biological tissue, and a downward direction will be referred to as downward therefrom. Further, a direction toward the viewer from Fig. 1B will be referred to as left direction, and a direction toward the back of the sheet will be referred to as right direction (in other words, a downward direction in Figs. 1A and 1C will be referred to as left direction, and an upward direction will be referred to as right direction). In Figs. 1A to 1C, a left direction may be referred to as front or distal side, and a right direction may be referred to as rear or proximal side.

The adhesive applicator for biological tissue 100 according to this embodiment shown in Figs. 1A to 1C is employed for applying medical fluids different in viscosity, for example fibrinogen solution and thrombin solution, to an affected part for bonding. Hereafter, the thrombin solution having relatively low viscosity may be referred to as first medical fluid, and the fibrinogen solution having relatively high viscosity may be referred to as second medical fluid.

The first medical fluid having the lower viscosity is loaded in a first syringe body 10a. The first medical fluid flows through a first medical fluid tube 5a and is dispensed from a first medical fluid outlet 4a. The second medical fluid having the higher viscosity is loaded in a second syringe body 10b. The second medical fluid flows through a second medical fluid tube 5b and is dispensed from a second medical fluid outlet 4b.

The adhesive applicator for biological tissue 100 includes a nozzle body 1, a gas inlet 2, a first and a second medical fluid inlets 3a, 3b, the first and the second medical fluid outlets 4a, 4b, the first and the second medical fluid tubes 5a, 5b, a gas outlet 6, communication paths 8a, 8b, and a check valve 7b. The syringe body 10a, the medical fluid inlet 3a, the medical fluid outlet 4a, and the medical fluid tube 5a constitute the flow path of the first medical fluid. The syringe body 10b, the medical fluid inlet 3b, the medical fluid outlet 4b, and the medical fluid tube 5b constitute the flow path of the second medical fluid. Hereafter, the ordinals "first" and "second" will be employed where it is necessary to clearly distinguish between the flow path of the first medical fluid and the flow path of the second medical fluid.

The nozzle body 1 includes therein a space 1c. The gas inlet 2 is used for loading therethrough a gas into the space 1c. The first and the second medical fluid inlets 3a, 3b are located inside the nozzle body 1. The first and the second medical fluid outlets 4a, 4b are located at the tip portion of the nozzle body 1. The first and the second medical fluid tubes 5a, 5b are arranged inside the nozzle body 1. Here, the expression "arranged inside the nozzle body" means that at least a part of the medical fluid tube is located inside the nozzle body.
The first medical fluid tube 5a communicates between the first medical fluid inlet 3a and the first medical fluid outlet 4a. The second medical fluid tube 5b communicates between the second medical fluid inlet 3b and the second medical fluid outlet 4b. The gas outlet 6 is located close to the first and the second medical fluid outlets 4a, 4b. The gas loaded in the nozzle body 1 is ejected through the gas outlet 6, to atomize the first and the second medical fluids respectively dispensed from the first and the second medical fluid outlets 4a, 4b and mix the medical fluids together.

The communication paths 8a, 8b are provided in the second medical fluid tube 5b through which the second medical fluid having the higher viscosity flows. This embodiment exemplifies the case where a plurality (two) of communication paths 8a, 8b is provided at different positions in the axial direction of the second medical fluid tube 5b. The communication paths 8a, 8b communicate between inside of the nozzle body 1 and inside of the second medical fluid tube 5b. The communication paths 8a, 8b serve to introduce the gas into the nozzle body 1 toward the distal end of the second medical fluid tube 5b (to the side of the second medical fluid outlet 4b).

The check valve 7b is located in the second medical fluid tube 5b, at a position upstream from the communication paths 8a, 8b. The expression "upstream from the communication paths 8a, 8b" means that the check valve 7b is located further upstream from one of the communication paths (in this embodiment, the communication path 8b) which is located at upstream from the other communication path. The check valve 7b only allows one-way flow of the second medical fluid from the second medical fluid inlet 3b into the second medical fluid tube 5b. To be more detailed, the check valve 7b only allows a liquid or gas to flow from the second medical fluid inlet 3b toward the second medical fluid outlet 4b, and inhibits the liquid or gas from reversely flowing from the side of the second medical fluid outlet 4b toward the second medical fluid inlet 3b. The check valve 7b may be located at the middle of the medical fluid tube 5b, between the medical fluid inlet 3b and the medical fluid tube 5b, or between the medical fluid inlet 3b and the syringe body 10b.

With the foregoing configuration, when the dispensation of the medical fluid is suspended during intermittent use of the adhesive applicator for biological tissue 100, the residue of the second medical fluid having the higher viscosity can be easily discharged out of the medical fluid outlet 4b with the pressure of the gas that flows into the second medical fluid tube 5b through the communication paths 8a, 8b. Such a configuration effectively prevents solidification of the medical fluid, thereby enabling smooth intermittent use of the adhesive applicator for biological tissue 100. In this embodiment, in addition, the check valve 7b is provided between the syringe body 10b and the communication paths 8a, 8b, more particularly between the syringe body 10b and the medical fluid inlet 3b located on the rear side of all the communication paths 8a, 8b. Therefore, the gas is prevented from flowing into the syringe body 10b, when the gas serves to discharge the medical fluid.

In this embodiment, the communication path is not provided in the first medical fluid tube 5a through which the first medical fluid having the lower viscosity flows, and only the second medical fluid tube 5b through which the second medical fluid having the higher viscosity flows includes the communication paths 8a, 8b. The first medical fluid having the lower viscosity exhibits higher fluidity in the first medical fluid tube 5a. Accordingly, the leading end of the first medical fluid retreats from the first medical fluid outlet 4a by a predetermined distance, after the dispensation is suspended. In the case where the gas continues to spout even after the physician stops pressing the syringe, a certain amount of medical fluid is still sucked out of the medical fluid tube by negative pressure generated by the gas. Therefore, the leading end of the medical fluid retreats from the medical fluid outlet after the dispensation is suspended. A larger amount of the first medical fluid, which is lower in viscosity, is sucked out compared with the second medical fluid, and hence the leading end of the first medical fluid retreats farther backward than the second medical fluid does, after the dispensation is suspended. For such reasons, it may be assumed that the first medical fluid tube 5a through which the first medical fluid flows is not likely to cause solidification at the first medical fluid outlet 4a, regardless of not including the communication paths 8a, 8b. In contrast, since the leading end of the second medical fluid having the higher viscosity is prone to be located close to the medical fluid outlet 4b after the dispensation is suspended, and therefore it is preferable to discharge the residual medical fluid with the high-pressure gas, as in this embodiment.

However, as a variation of this embodiment, not only the second medical fluid tube 5b but also the first medical fluid tube 5a may include the communication paths 8a, 8b. In this case, it is preferable to provide a check valve 7a upstream from the communication paths 8a, 8b in the flow path of the first medical fluid. Further, it is preferable that the aperture area of the communication paths 8a, 8b in the first medical fluid tube 5a be smaller than the aperture area of the communication paths 8a, 8b in the second medical fluid tube 5b. Such a configuration allows the residual medical fluid in the first medical fluid tube 5a (lower viscosity) and the residual medical fluid in the second medical fluid tube 5b (higher viscosity) to be discharged in an appropriate balance by the pressure of the gas. Here, even in the case where the communication paths 8a, 8b are not provided in the first medical fluid tube 5a as in this embodiment, it is still preferable to provide the check valve 7a between the first medical fluid tube 5a and a piston 12a, Such a configuration suppresses intrusion of bubbles into the piston 12a.

The adhesive applicator for biological tissue 100 according to this embodiment includes the first syringe body 10a connected to the first medical fluid inlet 3a, and the second syringe body 10b connected to the second medical fluid inlet 3b. The second medical fluid loaded in the second syringe body 10b has higher viscosity than the first medical fluid loaded in the first syringe body 10a. The adhesive applicator for biological tissue 100 includes identification members 9a, 9b, 11a, 11b, 14a, 14b that distinguish between the first medical fluid tube 5a and the second medical fluid tube 5b.

The adhesive applicator for biological tissue 100 also includes a first piston 12a used for introducing the first medical fluid from the first syringe body 10a into the first medical fluid tube 5a, and a second piston 12b used for introducing the second medical fluid from the second syringe body 10b into the second medical fluid tube 5b. The first and the second pistons 12a, 12b are pressing members for supplying the medical fluids loaded in the syringe bodies 10a, 10b into the respective medical fluid tubes 5a, 5b.

The adhesive applicator for biological tissue 100 includes a piston joint member 13 connecting between the first and the second pistons 12a, 12b. The piston joint member 13 allows the pistons 12a, 12b to be pressed at the same time. Such a configuration enables the first and the second medical fluid in the medical fluid tubes 5a, 5b, different from each other in viscosity, to be dispensed from the medical fluid outlets 4a, 4b in a properly balanced ratio onto an affected part.

The adhesive applicator for biological tissue 100 further includes a gas supply tube 15 connected to the gas inlet 2 for supplying the gas into the nozzle body 1, and a filter 16 provided upstream from the gas inlet 2 for filtering the gas.

In addition, the adhesive applicator for biological tissue 100 includes a syringe body joint member 20 that combines the first and the second syringe bodies 10a, 10b, and a band 14 serving as a fixing member that fixes the nozzle body 1 and the syringe body joint member 20 together.

The identification members 9a, 9b are provided in the syringe bodies 10a, 10b. To be more detailed, the identification member 9a is provided on the first piston 12a, and the identification member 9b is provided on the second piston 12b. The identification members 11a, 11b are provided in the vicinity of the medical fluid inlets 3a, 3b. The identification members 14a, 14b are provided on the band 14. The identification members 14a, 14b, the identification members 11a, 11b, and the identification members 9a, 9b each constitute a pair.

Hereunder, the nozzle body 1 according to this embodiment will be described in details. As shown in Figs. 1A to 1C, the nozzle body 1 includes a lid 1a air-tightly connected to the rear end of the nozzle body 1. A support pole 1b is formed on the wall of the lid 1a, so as to project in a forward direction. As shown in Fig. 1C, the support pole 1b extends to reach the tip portion of the nozzle body 1. The support pole 1b serves to support the nozzle body 1 and the medical fluid outlets 4a, 4b, to increase resistance against spray-mixing pressure of the medical fluid and the gas. A gap is defined between the outer circumferential surface of the support pole 1b and the inner circumferential surface of the nozzle body 1, and between the former and the outer circumferential surface of the medical fluid outlets 4a, 4b. This gap constitutes the gas outlet 6. The gas outlet 6 is located between the two medical fluid outlets 4a, 4b and on the rear side thereof, so that the first and the second medical fluids are properly sprayed and mixed. In addition, as shown in Fig. 1C, the gas inlet 2, through which the high-pressure gas for spraying the medical fluid is introduced into the nozzle body 1, is formed as an opening on the bottom wall of the nozzle body 1. Upon ejecting the gas at a high speed through the gas outlet 6, negative pressure is generated in the vicinity of the gas outlet 6, and the medical fluids are drawn toward the medical fluid outlets 4a, 4b. Accordingly, the medical fluids can be smoothly dispensed by pressing the pistons 12a, 12b only with a small force. The first and the second medical fluids thus dispensed are atomized and mixed together by the ejection of the gas.

As shown in Fig. 1B, the gas supply tube 15 communicating with a gas supply source such as a gas tank (not shown) is formed so as to project from the gas inlet 2 of the nozzle body 1. In addition, the filter 16 for filtering the gas is connected to the upstream side of the gas supply tube 15, and hence the gas inlet 2 communicates with the gas supply source through the filter 16. Accordingly, the filter 16 removes impurities such as dust contained in the gas, to thereby supply the purified gas into the space 1c in the nozzle body 1. As a result, more hygienic medical fluids can be applied to an affected part. Further, when the dispensation of the medical fluid is suspended and the gas flows into the medical fluid tube 5b through the communication paths 8a, 8b to discharge the medical fluid, clogging of the medical fluid tube 5b due to dust or the like can be prevented, which further assures the smooth flow and dispensation of the medical fluid.

Referring to Figs. 4 and 5A, 5B, the lid 1a of the nozzle body 1 includes communication orifices 1e1, 1e2, for communication between the medical fluid tubes 5a, 5b and the syringe bodies 10a, 10b, respectively. The lid 1a also includes tube connection bases 1d1, 1d2 projecting forward in a cylindrical shape from the positions respectively corresponding to the communication orifices 1e1, 1e2, for liquid-tight insertion and connection of the medical fluid tubes 5a, 5b. Further, valve attaching portions 1f1, 1f2 of a cylindrical shape for mounting the check valves 7a, 7b therein are formed on the rear face of the lid 1a, at the positions respectively corresponding to the communication orifices 1e1, 1e2. To the valve attaching portions 1f1, 1f2, double cylindrical portions 19c, 19d are connected, which are formed so as to project from support members 19a, 19b that support the check valves 7a, 7b mounted in the valve attaching portions 1f1, 1f2. Around the outer circumference of the support members 19a, 19b, support member covers 21a; 21b are attached. The support member covers 21a, 21b are finished in red and blue respectively, to enable identification of the syringe bodies 10a, 10b when setting the syringes, as will be subsequently described. The support member covers 21a, 21b also serve to suppress the stepped cylindrical portions 19c, 19d from expanding outward, to thereby prevent the support members 19a, 19b from falling off and improve air-tightness and liquid-tightness.

The nozzle body 1 may be formed of a semi-transparent or opaque material that provides a light shielding effect, or of a transparent material that allows easy visual confirmation of the content, depending on the nature and purpose of the medical fluid. Here, it is preferable to employ a medical plastic material for the components of the adhesive applicator for biological tissue according to this embodiment, such as the nozzle body 1, the medical fluid outlets 4a, 4b, the medical fluid tubes 5a, 5b, the gas supply tube 15, and the check valves 7a, 7b. However, the present invention is not limited to such materials, and different materials for medical purposes may be employed. Further, it is preferable to employ a material that keeps the medical fluid unaffected.

The check valves 7a, 7b will now be described hereunder. As described above, the check valves 7a, 7b are mounted in the cylindrical valve attaching portions 1f1, 1f2 as shown in Fig. 1C which shows a general view and Fig. 4 whish shows an enlarged view. As is apparent in Fig. 4 showing an enlarged view in the vicinity of the valve 7b, the check valves 7a, 7b are so-called duckbill valves that can be opened by applying pressure to the inner face and closed by applying pressure to the outer face. Accordingly, when the medical fluid is to be dispensed the valve element is opened, so as to allow the medical fluid to flow into the medical fluid tube 5b through the medical fluid inlet 3b. In contrast, when the dispensation of the medical fluid is suspended, the gas pressure is applied in the direction from inside the medical fluid tube 5b toward the medical fluid inlet 3b, and hence the valve element is closed to restrict reverse flow of the medical fluid and intrusion of the gas from inside the medical fluid tube 5b to the syringe body 10b. Although not shown, the check valve 7a has the same configuration as that of the check valve 7b, and provides the same effect.

The syringe bodies 10a, 10b include the identification members 9a, 9b for distinction of the medical fluid to be loaded. In this embodiment, the syringe body 10a is formed of a transparent material and the piston 12a that squeezes the medical fluid is finished in red and the piston 12b inserted in the syringe body 10b is finished in blue, to respectively serve as the identification members 9a, 9b. Further, an identification sticker 11, on which a red mark (identification member 11a) and a blue mark (identification member 11b) are printed, is adhered on the nozzle body 1 connected to the syringe bodies 10a, 10b identified by the red and blue pistons 12a, 12b, as shown in Fig. 1A. In addition, also as shown in Fig. 1A, the band 14 serving as the fixing member for fixing the nozzle body 1 and the two syringe bodies 10a, 10b together includes the identification members. More specifically, the letter "RED" and an arrow indicating the attaching position of the syringe body 10a, serving as the identification member 14a, are carved in relief on the band 14. Likewise, the letter "BLUE" and an arrow indicating the attaching position of the syringe body 10b, serving as the identification member 14b, are carved in relief on the band 14. These identification members ensure that the syringe bodies 10a, 10b are placed in correct positions for connection to the nozzle body 1. Further, as described above, the support member covers 21a, 21b are also finished in red and blue respectively, thus allowing identification. Such a configuration further assures that the two types of syringe bodies 10a, 10b are correctly connected to the nozzle body 1.

Hereunder, a method of combining the nozzle body 1 and the syringe bodies 10a, 10b with the band 14 will be described. As shown in Fig. 1A showing a general view and Fig. 2 showing a perspective view, the front end portion of the band 14 is inserted and fixed in a slot 1g formed on the rear end portion of the nozzle body 1. The band 14 includes a through hole 14c opened on the rear end portion thereof. On the other hand, the syringe body joint member 20 for combining the syringe bodies 10a, 10b includes a projection 20a projecting upward to be inserted in the through hole 14c at front end portion of the syringe body joint member 20. After the syringe bodies 10a, 10b are respectively inserted in the support members 19a, 19b on the side of the nozzle body 1, the band 14 is pressed down so as to insert the projection 20a of the syringe body joint member 20 into the through hole 14c. Here, the band 14 includes a pressing portion 14d projecting obliquely upward from the rear end portion to be pressed by a finger, for facilitating the action of pressing down the band 14 and inserting the projection 20a. The band 14 serves to prevent the syringe bodies 10a, 10b from being disengaged from the nozzle body 1, and to restrict the syringe bodies 10a, 10b from moving back and forth and up and downward.

The aforementioned configuration stabilizes the pressing action of the pistons 12a, 12b to thereby allow the medical fluid to be smoothly dispensed, thus allowing stabilized use of the adhesive applicator for biological tissue 100. Further, as already described, the band 14 includes the identification member 14a composed of the letter "RED" and the arrow indicating the position of the syringe body 10a, and the identification member 14b composed of the letter "BLUE" and the arrow indicating the position of the syringe body 10b. Therefore, erroneous connection of the syringe bodies 10a, 10b can be more securely prevented. Here, although the letters "RED" and "BLUE" are employed in this embodiment, the present invention is not limited to those letters. The respective initials R and B of red and blue may be employed. Further, without limitation to red and blue, desired colors may be adopted according to the color of the syringe bodies 10a, 10b and the type of the medical fluid.

Hereunder, the communication paths 8a, 8b in the medical fluid tube 5b according to this embodiment will be described in details. Fig. 3A is a schematic perspective view of the medical fluid tube 5b, and Fig. 3B is a cross-sectional view taken along a line B-B in Fig. 3A. The communication paths 8a, 8b are slits formed in the wall of the medical fluid tube 5b. As shown in Fig. 3B, the depthwise direction of the slits is inwardly inclined toward the distal end portion of the medical fluid tube 5b (the side of the medical fluid outlet 4b).

As shown in Fig. 3B, the communication paths 8a, 8b (slits) have an opening width w1 on the outer surface of the medical fluid tube 5b, which is wider than an opening width w2 on the inner surface of the tube wall. The communication paths 8a, 8b are formed by cutting the wall of the medical fluid tube 5b in a direction intersecting the axial direction of the medical fluid tube 5b, with a radially inward inclination from the upstream side (right side in Fig. 3B) toward the downstream side (left side in Fig. 3B). The two communication paths 8a, 8b are oriented parallel to each other in the axial (longitudinal) direction. When the medical fluid is dispensed as shown in Fig. 5A, the dispensation pressure which is higher than the gas pressure is applied to the medical fluid, and hence the gas is kept from intruding in the medical fluid tube 5b through the communication paths 8a, 8b. Therefore, the flow of the medical fluid from the medical fluid outlet 4b is not affected by the communication paths 8a, 8b, and the medical fluid can be smoothly dispensed.

The medical fluid tubes 5a, 5b according to this embodiment are formed of a soft plastic tube. Accordingly, the slit-shaped cuttings that serve as the communication paths 8a, 8b can be easily formed. The plastic material is not specifically limited, but it is preferable to employ, for example, a polyvinyl chloride resin, a polyurethane resin, or a silicone resin. Here, although the medical fluid tubes 5a, 5b are formed of a soft plastic material in this embodiment, different materials such as a metal may be employed instead.

Further, when the dispensation of the medical fluid is suspended as shown in Fig. 5B, the communication paths 8a, 8b are opened by the pressure of the gas loaded in the space 1c in the nozzle body 1. Then the gas flows into the medical fluid tube 5b through the communication paths 8a, 8b and outwardly discharges the medical fluid remaining downstream from the communication paths 8a, 8b. Such an arrangement restricts the medical fluid in the medical fluid tube 5b from contacting the ambient air and the counterpart medical fluid, thereby preventing clogging in the downstream portion of the medical fluid tube 5b, even though a certain period of time elapses before the next dispensation of the medical fluid.

To apply the medical fluid to an affected part of an organism by spraying with the adhesive applicator for biological tissue 100 configured as above, first the syringe bodies 10a, 10b are connected to the nozzle body 1. When doing so, the syringe body 10b loaded with the medical fluid having the higher viscosity (for example, fibrinogen solution) is connected to the second medical fluid tube 5b having the communication paths 8a, 8b. Likewise, the syringe body 10a loaded with the medical fluid having the lower viscosity (for example, thrombin solution) is connected to the first medical fluid tube 5a without the communication path. As described above, the pistons 12a, 12b inserted in the syringe bodies 10a, 10b have the identification members 9a, 9b, the nozzle body 1 has the identification members 11a, 11b, and the band 14 has the identification members 14a, 14b. Accordingly, the syringe bodies 10a, 10b can be correctly set in place. Upon connecting the syringe bodies 10a, 10b, the pressing portion 14d of the band 14 is pressed downward so as to insert the projection 20a of the syringe body joint member 20 into the through hole 14c, thus to fix the syringe bodies 10a, 10b and the nozzle body 1 together.

Once the syringe bodies 10a, 10b are thus combined with the nozzle body 1, the syringe bodies 10a, 10b are restricted from wobbling in the axial and up/downward directions during the spraying of the medical fluids, and therefore the spraying can be smoothly performed. Then the gas is loaded in the space 1c in the nozzle body 1 by operating a button or the like (not shown), and ejected through the gas outlet 6. At the same time, the piston joint member 13 connecting the pistons 12a, 12b is pressed to squeeze the medical fluids into the medical fluid tubes 5a, 5b, and to dispense the medical fluids through the medical fluid outlets 4a, 4b, respectively. The medical fluids dispensed from the medical fluid outlets 4a, 4b are atomized and mixed together by the gas ejected from the gas outlet 6 as shown in Fig. 5A, and sprayed over the affected part. To spray the medical fluids, the pistons 12a, 12b can be simultaneously pressed with the piston joint member 13. Therefore, the two medical fluids can be smoothly dispensed and applied to the affected part in a properly balanced mixing ratio, which results in improved bonding effect.

When the dispensation of the medical fluid is temporarily suspended thereafter, the gas flows into the medical fluid tube 5b through the communication paths 8a, 8b because of the gas pressure, and outwardly discharges the medical fluid remaining downstream from the communication paths 8a, 8b, through the medical fluid outlet 4b. Accordingly, the medical fluid in the medical fluid tube 5b is restricted from contacting the ambient air, and also from being mixed with the medical fluid in the medical fluid tube 5a, and therefore solidification of the medical fluid at the medical fluid outlets 4a, 4b can be effectively prevented. Therefore, the next dispensation of the medical fluids can also be smoothly performed.

Further, when the medical fluid is discharged from the medical fluid tube 5b, the check valve 7b serves to suppress the gas pressure from being imposed on the supply side of the medical fluid. Here, bubbles may be mixed in the medical fluid during the preparation thereof, and the bubbles may remain inside the syringe body. In an applicator in which a check valve is not provided between a communication path and a medical fluid inlet as the technique according to the patent document 2, both of the gas pressure and the squeezing force of the piston in the syringe body are imposed on the bubbles, so as to compress the bubbles. When the medical fluid is released from the squeezing force of the piston and stops being dispensed, the compressed bubbles elastically restore the original volume gradually. As a result, the leading end of the medical fluid may accidentally advance in the medical fluid tube from which the residual medical fluid has once been removed, and may thus solidify. In this embodiment, in contrast, the check valve 7b is provided upstream from the communication paths 8a, 8b, more particularly at the rear end of the medical fluid tube 5b, and hence the bubbles are exempted from being subjected to the gas pressure. Accordingly, the bubbles mixed in the syringe body in advance are prevented from being compressed by the gas pressure. Therefore the elastic restoration of the volume of the bubbles barely takes place and the accidental advance of the leading end of the medical fluid can be suppressed. The check valve 7b can thus enhance the prevention of the solidification, without compromising the discharging effect of the medical fluid from the medical fluid tubes 5a, 5b through the communication paths 8a, 8b. Further, the applicator with excellent capability of preventing the solidification of the medical fluid can be easily manufactured with a minimized number of parts, simply by providing the communication path and the check valve.

### Second Embodiment

Figs. 6A to 6D are enlarged fragmentary drawings of a medical fluid tube to which a generally square tube-shaped communication tubes are respectively connected to slit-shaped communication paths, in an adhesive applicator for biological tissue according to a second embodiment. Fig. 6A is a cross-sectional view of the medical fluid tube 105b taken in the axial direction, and Fig. 6B is a plan view of Fig. 6A.

The communication paths 108a, 108b according to this embodiment are gas flow paths longer than the wall thickness of the medical fluid tube 105b. Here, the wall thickness refers to the average thickness of the wall of the medical fluid tube 105b except for the positions corresponding to the communication paths 108a, 108b. These gas flow paths are constituted of communication tubes 108c, 108d. The depthwise direction of the gas flow path is inwardly inclined toward the distal end portion of the medical fluid tube 105b (left side in Fig. 6A). The communication paths 108a, 108b according to this embodiment can conduct the gas in the nozzle body into the communication paths 108a, 108b.

The communication tubes 108c, 108d according to this embodiment are formed so as to inwardly project from the inner wall surface of the medical fluid tube 105b. Various modifications may be made to this embodiment. Fig. 6C is a cross-sectional view of a medical fluid tube 115b, to an outer surface of which communication tubes 118c, 118d inclined from the upstream side (right side in Fig. 6C) toward the downstream side (left side in Fig. 6C) are connected. Fig. 6D is a plan view of Fig. 6C.

In this embodiment, the medical fluid tube 105b (115b) includes the communication tubes 108c, 108d (118c, 118d) inclined from the side of the medical fluid inlet 3b toward the medical fluid outlet 4b, as shown in Figs. 6A to 6D. Orienting the gas flow path toward the medical fluid outlet 4b allows the gas to be surely guided toward the medical fluid outlet 4b when the dispensation of the medical fluid is suspended, to thereby smoothly discharge the medical fluid despite the high viscosity. In addition, by increasing the wall thickness at the base portion of the communication tubes 108c, 108d (118c, 118d) compared with the remaining portion of the medical fluid tube 105b (115b) as shown in Figs. 6A to 6D, the communication paths 108a, 108b (118a, 118b) can be prevented from being depressed and closed by the gas pressure when the gas is about to flow into the medical fluid tube 105b (115b). The medical fluid tube 105b (115b) according to this embodiment may be formed of a soft plastic or a metal, by a known method such as molding or pultrusion.

### Third Embodiment

Figs. 7A to 7D are enlarged fragmentary drawings of a medical fluid tube to which a generally cylindrical communication tubes are respectively connected to generally circular communication paths, in an adhesive applicator for biological tissue according to a third embodiment. Fig. 7A is a cross-sectional view of a medical fluid tube 205b where communication tubes 208c, 208d, serving as gas flow paths,are connected to the inner side of communication paths 208a, 208b, the communication tubes 208c, 208d being inclined from the side of the medical fluid inlet 3b toward the medical fluid outlet 4b so as to guide the gas in the nozzle body 1 toward the medical fluid outlet 4b. Fig. 7B is a plan view of Fig. 7A. Various modifications may be made to this embodiment. For example, Fig. 7C is a cross-sectional view of a medical fluid tube 215b where communication tubes 218c, 218d inclined from the upstream side toward the downstream side are connected to the outer side of communication paths 218a, 218b. Fig. 7D is a plan view of Fig. 7C.

Orienting the gas flow path toward the medical fluid outlet 4b by providing the communication tubes 208c, 208d (218c, 218d) allows the gas to be surely guided toward the medical fluid outlet 4b when the dispensation of the medical fluid is suspended, to thereby smoothly discharge the medical fluid despite the high viscosity. In addition, by increasing the wall thickness at the base portion of the communication tubes 208c, 248d (218c, 218d) compared with the remaining portion of the medical fluid tube 205b (215b) as shown in Figs. 7A to 7D, the communication paths 208a, 208b (218a, 218b) can be prevented from being depressed and closed by the gas pressure when the gas is about to flow into the medical fluid tube 205b (215b). The medical fluid tube 205b (215b) according to this embodiment may be formed of a soft plastic or a metal, by a known method such as molding or pultrusion.

Although two medical fluid tubes are employed in the foregoing embodiments, three or more medical fluid tubes may be employed to dispense three or more types of medical fluids. Further, the medical fluid tubes may respectively include different numbers of communication paths, for example according to the viscosity of the medical fluids to be dispensed, and the syringe body loaded with the medical fluid having a higher viscosity may be connected to the medical fluid tube including the communication paths having a larger total aperture area in order of decreasing the viscosity and decreasing the total aperture area. Such an arrangement allows the medical fluids to be dispensed in an appropriate balance according to the viscosity.

Although the communication paths according to the foregoing embodiments are configured so as to restrict the gas from intruding through the communication paths while the medical fluid is being dispensed, the gas may be allowed to intrude through the communication paths even while the medical fluid is being dispensed. In particular, in the case of dispensing medical fluids that are different in viscosity, the dispensation of the medical fluid having the higher viscosity can be facilitated by introducing such medical fluid into the medical fluid tube with the communication paths that allow the gas to intrude, because of the pressure of the gas that flows into the medical fluid tube. Therefore, the force necessary for pressing the syringe bodies respectively loaded with the medical fluid having the higher viscosity and the medical fluid having the lower viscosity is leveled off, which further facilitates the medical fluids to be dispensed in a properly balanced ratio. The applicator thus configured is broadly applicable to medical fluids of various levels of viscosity.

Although a plurality of medical fluid tubes 5a, 5b is accommodated in a single nozzle body 1 in the foregoing embodiment, the present invention is not limited to such a configuration. For example, a plurality of nozzle bodies each having a space therein may be respectively connected to the plurality of medical fluid tubes. In other words, the adhesive applicator for biological tissue may include a plurality of nozzle bodies, unlike the foregoing embodiment. In this case, the nozzle bodies each include, though not illustrated, the medical fluid inlet, the medical fluid outlet, the medical fluid tube communicating therebetween, the gas inlet, and the gas outlet. The medical fluid tube in one of the nozzle bodies includes the communication path and the check valve located upstream from the communication path. In the adhesive applicator for biological tissue having a plurality of nozzle bodies also, the residual medical fluid can be discharged outside by the pressure of the gas that intrudes through the communication path, when the dispensation of the medical fluid is suspended. Such a configuration also effectively prevents the solidification of the medical fluid, thereby enabling the adhesive applicator for biological tissue to be intermittently operated smoothly.

Although each of the plurality of nozzle bodies includes a single medical fluid tube in the foregoing variation of the embodiments, the present invention is not limited to such a configuration. One or a plurality of nozzle bodies may include a plurality of medical fluid tubes. In this case, a medical fluid tube with the communication path and a medical fluid tube without the communication path may be provided in any of the nozzle bodies, or all the medical fluid tubes in any of the nozzle bodies may include the communication path. Whichever the case may be, providing the check valve also in the medical fluid tube without the communication path, in addition to the medical fluid tube with the communication path, prevents reverse flow of the medical fluid or the gas from the medical fluid tube into the syringe body caused by the return pressure generated upon suspending the dispensation of the medical fluid. Thus, as long as the applicator includes a medical fluid tube having both the communication path and the check valve, other medical fluid tubes may be configured as desired, as to whether the communication path or the check valve is to be provided.

It is a matter of course that the present invention is in no way limited to any of the foregoing embodiments, and various modifications may be made within the scope and spirit of the present invention.

The foregoing embodiments encompass the following technical ideas.
(1) An adhesive applicator for biological tissue, including a nozzle body having a space therein; a gas inlet through which gas is introduced into the space inside the nozzle body; a plurality of medical fluid inlets provided to the nozzle body; a plurality of medical fluid outlets provided at a distal end portion of the nozzle body; a plurality of medical fluid tubes arranged inside the nozzle body and respectively communicating between the plurality of medical fluid inlets and the plurality of medical fluid outlets; a gas outlet located close to the medical fluid outlets and configured to eject the gas loaded through the gas inlet in the nozzle body, to thereby atomize the medical fluids dispensed through the medical fluid outlets and mix the medical fluids together; at least one communication path formed in at least one of the medical fluid tubes and communicating between inside of the nozzle body and inside of the medical fluid tube to conduct the gas in the nozzle body toward the medical fluid outlet; and a check valve provided at least in the medical fluid tube that includes the communication path and configured to only allow one-way flow of the medical fluid from the medical fluid inlet into the medical fluid tube and to restrict the medical fluid from flowing toward the medical fluid inlet from the inside of the medical fluid tube.
(2) The adhesive applicator for biological tissue according to (1) above, in which the medical fluid tube includes a plurality of the communication paths aligned in the longitudinal direction of the tube wall.
(3) The adhesive applicator for biological tissue according to (1) or (2) above, in which the communication paths of each of the medical fluid tubes have different values of total aperture area.
(4) The adhesive applicator for biological tissue according to any of (1) to (3) above, further including a plurality of syringe bodies respectively loaded with the medical fluids that are different in viscosity, and respectively connected to the plurality of medical fluid inlets.
(5) The adhesive applicator for biological tissue according to (4) above, in which the syringe body loaded with the medical fluid having higher viscosity is connected to the medical fluid tube that includes the communication path having larger total aperture area.
(6) The adhesive applicator for biological tissue according to (4) or (5) above, further including an identification member, for distinction between the medical fluid tubes to which the syringe bodies loaded with the medical fluids different in viscosity are to be connected.
(7) The adhesive applicator for biological tissue according to any of (4) to (6) above, in which the plurality of syringe bodies each further includes a pressing member for introducing the medical fluid loaded in the syringe body into the corresponding medical fluid tube, and a pressing member joint member that combines the plurality of pressing members to enable the pressing members to be simultaneously pressed.
(8) The adhesive applicator for biological tissue according to (7) above, further including a syringe body joint member that combines the plurality of syringe bodies, and a fixing member that fixes the nozzle body and the syringe body joint member together.
(9) The adhesive applicator for biological tissue according to any of (1) to (8) above, in which the communication path is formed in a slit shape so as to extend on a tube wall of the medical fluid tube in a direction intersecting the axial direction of the medical fluid tube.
(10) The adhesive applicator for biological tissue according to (9) above, in which the slit-shaped communication path is formed by cutting the tube wall such that the side-view shape of the communication path is inclined toward the medical fluid outlet from the side of the medical fluid inlet, and that the opening on the outer surface of the tube is larger than the opening on the inner surface of the tube wall.
(11) The adhesive applicator for biological tissue according to any of (1) to (10) above, in which the communication path includes a communication tube, formed on the outer surface or inner wall surface of the medical fluid tube, having a gas flow path with an inclination toward the medical fluid outlet from the side of the medical fluid inlet, so as to conduct the gas inside the nozzle body toward the medical fluid outlet.
(12) The adhesive applicator for biological tissue according to any of (1) to (11) above, in which the medical fluid tube is formed of a soft plastic tube.
(13) The adhesive applicator for biological tissue according to any of (1) to (12) above, in which a plurality of the nozzle bodies each having a space therein is respectively provided for a plurality of the medical fluid tubes.

This application claims priority based on Japanese Patent Application No. 2010-251515 filed on November 10, 2010, the entire content of which is incorporated hereinto.

## Claims

1. An adhesive applicator for biological tissue, comprising:
a nozzle body having a space therein;
a gas inlet through which gas is introduced into the space;
a first medical fluid inlet and a second medical fluid inlet provided to the nozzle body;
a first medical fluid outlet and a second medical fluid outlet provided at a distal end portion of the nozzle body;
a first medical fluid tube communicating between the first medical fluid inlet and the first medical fluid outlet, and a second medical fluid tube communicating between the second medical fluid inlet and the second medical fluid outlet, the first and the second medical fluid tubes being arranged inside the nozzle body;
a gas outlet located close to the first and the second medical fluid outlets and configured to eject the gas loaded in the nozzle body to thereby atomize the medical fluids respectively dispensed through the first and the second medical fluid outlets and mix the medical fluids together;
a communication path formed in the second medical fluid tube for communication between inside of the nozzle body and inside of the second medical fluid tube; and
a check valve provided upstream from the communication path and configured to only allow one-way flow of the medical fluid from the second medical fluid inlet into the second medical fluid tube.

2. The adhesive applicator for biological tissue according to claim 1,
wherein the second medical fluid tube includes a plurality of the communication paths formed at different positions in an axial direction of the second medical fluid tube.

3. The adhesive applicator for biological tissue according to claim 1 or 2,
wherein the first medical fluid tube includes a communication path communicating between the inside of the nozzle body and inside of the first medical fluid tube, and
an aperture area of the communication path formed in the first medical fluid tube is smaller than an aperture area of the communication path formed in the second medical fluid tube.

4. The adhesive applicator for biological tissue according to claim 1 or 2,
wherein the first medical fluid tube is without the communication path, and only the second medical fluid tube includes the communication path.

5. The adhesive applicator for biological tissue according to claim 3 or 4, further comprising a first syringe body connected to the first medical fluid inlet and a second syringe body connected to the second medical fluid inlet,
wherein the second medical fluid loaded in the second syringe body has higher viscosity than the first medical fluid loaded in the first syringe body.

6. The adhesive applicator for biological tissue according to claim 5, further comprising an identification member for distinction between the first medical fluid tube and the second medical fluid tube.

7. The adhesive applicator for biological tissue according to claim 5 or 6, further comprising:
a first piston that introduces the first medical fluid from the first syringe body into the first medical fluid tube;
a second piston that introduces the second medical fluid from the second syringe body into the second medical fluid tube; and
a joint member connecting the first piston and the second piston.

8. The adhesive applicator for biological tissue according to claim 7, further comprising:
a syringe body joint member that combines the first syringe body and the second syringe body; and
a fixing member that fixes the nozzle body and the syringe body joint member together.

9. The adhesive applicator for biological tissue according to any one of claims 1 to 8,
wherein the communication path is a slit formed in a tube wall of the medical fluid tube, and a depthwise direction of the slit is inwardly inclined toward a distal end portion of the medical fluid tube.

10. The adhesive applicator for biological tissue according to claim 9,
wherein the opening width of the slit on the outer surface of the tube is larger than the opening width of the slit on the inner surface of the tube wall.

11. The adhesive applicator for biological tissue according to any one of claims 1 to 10,
wherein the communication path serves as a gas flow path having a length longer than a wall thickness of the medical fluid tube, and a depthwise direction of the gas flow path is inwardly inclined toward a distal end portion of the medical fluid tube.

12. The adhesive applicator for biological tissue according to any one of claims 1 to 11,
wherein the medical fluid tube is formed of a soft plastic tube.

13. The adhesive applicator for biological tissue according to any one of claims 1 to 12,
wherein a plurality of the nozzle bodies each having a space therein is respectively provided for a plurality of the medical fluid tubes.
